# EUROPEAN PATENT SPECIFICATION

(11) **EP 2 290 394 B1**
(45) Date of publication and mention of the grant of the patent: **08.11.2017**
(21) Application number: 10159440.6
(22) Date of filing: 09.04.2010
(51) Int. Cl.: G01S 7/52, G01S 15/89

(54) **Adaptively Setting a Transmit Frequency in an Ultrasound System**
Adaptive Einstellung einer Sendefrequenz in einem Ultraschallsystem
Réglage adaptatif d'une fréquence de émission dans un système d'ultrasons

(30) Priority: 17.08.2009 KR 20090075724
(43) Date of publication of application: 02.03.2011
(73) Proprietor: Samsung Medison Co., Ltd., Hongcheon-gun, Gangwon-do 25108 (KR)
(72) Inventor: Shin, Dong Kuk, Seoul 135-851 (KR); Kim, Jong Sik, Seoul 135-851 (KR)
(74) Representative: Schmid, Wolfgang

(56) References cited:
- EP-A2- 0 464 440
- WO-A1-96/03918
- WO-A2-02/47533

## Description

### TECHNICAL FIELD

The present disclosure generally relates to ultrasound systems, and more particularly to adaptively setting a transmit frequency in an ultrasound system.

### BACKGROUND

An ultrasound system has become an important and popular diagnostic tool since it has a wide range of applications. Specifically, due to its non-invasive and non-destructive nature, the ultrasound system has been extensively used in the medical profession. Modern high-performance ultrasound systems and techniques are commonly used to produce two or three-dimensional diagnostic images of internal features of a target object (e.g., human organs).

The ultrasound system can transmit and receive ultrasound signals to and from a target object to thereby form 2D (two-dimensional) or 3D (three-dimensional) ultrasound images of the target object. Also, the ultrasound system can form an ultrasound image of the target object by using the receive signals relating to an image depth of the target object and a steering angle of scan-lines. The image depth represents the range of an ultrasound image of the target object, which is to be formed in a depth direction.

The WO 96/03918 A1 discloses a method for scanning a field of view using a scan format, wherein the carrier frequency of the imaging pulse is higher in the center of the field of view than at the edges. The frequency variation can be accomplished on transmit by modulating appropriately delayed programmable initial waveform information samples with a programmable carrier frequency. This results in a pulse transmitted into the body whose frequency is highest in the center portion of the scan, and is reduced in a controlled fashion as the steering angle is increased in order to mitigate grating lobe artifacts. The technique preserves signal energy because modulation merely translates the signal in frequency substantially without modification of the pulse shape itself. The technique is also useful on receive, wherein demodulation to or near baseband followed by post-beamformation predetection remodulation can correct for systematic scan-line-to-scan-line phase variations to ensure scan-line-to-scan-line phase coherency for subsequent coherent processing across scan lines or for coherent image formation using the phase and amplitude information from multiple beams.

The WO 02/47533 A2 discloses an ultrasound system that generates compound images from component frames having decorrelated speckle patterns. Successive sets of distinct, speckle-affecting parameters are used to generate successive component frames for compounding, and are selected such that the successive component frames have decorrelated speckle patterns. The speckle-affecting parameters that are changed from frame to frame may be selected from wide variety of parameters, including transmit beamformer parameters, receive beamformer parameters, and demodulator parameters. According to a preferred embodiment, the successive sets of speckle-affecting parameters differ from each other by at least two speckle-affecting parameters. According to another preferred embodiment, the amount by which each of the multiple speckle-affecting parameters is changed is less than a decorrelation threshold for that parameter, that is, by less than the amount that speckle-affecting parameter alone would be required to change in order to yield decorrelated speckle patterns if no other parameters were changed. When more speckle-affecting parameters are changed, each speckle-affecting parameter can be changed by an amount less than its decorrelation threshold and yet decorrelated speckle patterns can still be obtained. Moreover, because two different types of speckle-affecting parameters tend to alter the spatial resolution of the component frames in different ways, the spatial resolution of the compounded image can be better as compared to the scenario in which only one speckle-affecting parameter is altered by its decorrelation threshold.

The EP 0 464 440 A2 discloses an ultrasound system steps focus and power through a series of focal zones to provide real-time seamless images with a range exceeding the depth of field of the employed ultrasound transducer. The information required for the focus and power functions are stored in memories which are read out by a zone sequencer. The data is stored in registers in the drive circuitry so that no time is lost in generating succeeding power bursts. Echoes are received from successive zones at a given steering position rather than at successive steering positions of a given zone. Provision is also made for adapting time-varying gain functions applied at a receiver as a function of zone to optimize contrast uniformity and minimize contrast discontinuities. The zone sequencer controls the input of processed echo signals into a video buffer, gating out unwanted information and splicing echo signal segments from successive zones into continuous sector lines for display on a monitor. Echoes are gathered from all zones at each steering position rather than at all steering positions for each zone so as to minimize temporal discontinuities in the constructed image.

### SUMMARY

Embodiments for adaptively setting a transmit frequency in an ultrasound system are disclosed herein. In one embodiment, there is provided an ultrasound system according to claim 1. In another embodiment, there is provided a method according to claim 2. In yet another embodiment, there is provided a computer readable medium according to claim 3.

### BRIEF DESCRIPTION OF THE DRAWINGS

Figure 1 is a block diagram showing an illustrative embodiment of an ultrasound system.
Figure 2 is a graph showing an example of a grating lobe according to transmit (Tx) frequencies.
Figure 3 is a graph showing an example of optimal Tx frequencies set according to image depths.
Figure 4 is a schematic diagram showing an example of steering angles set according to the image depths.
Figure 5 is a graph showing an example of optimal Tx frequencies set according to steering angles.
Figure 6 is a schematic diagram showing an example of the optimal Tx frequencies set according to the image depths and steering angles.
Figure 7 is a block diagram showing an illustrative embodiment of an ultrasound data acquisition unit.

### DETAILED DESCRIPTION

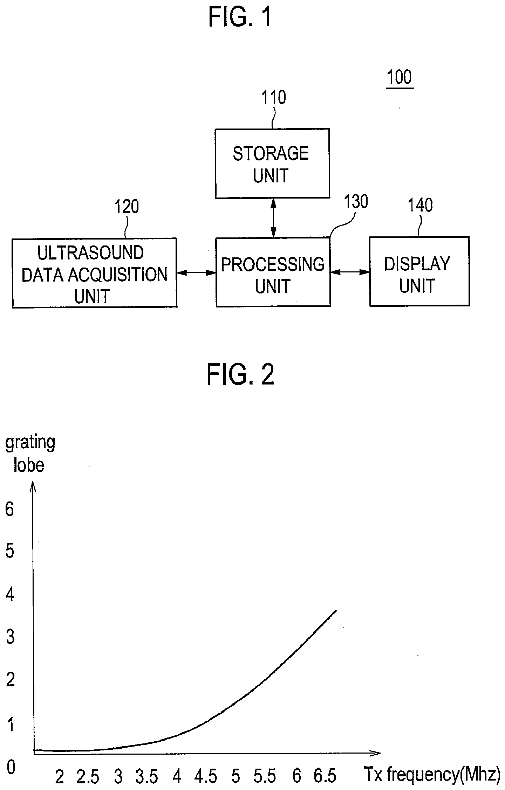

A detailed description is provided with reference to the accompanying drawings. One of ordinary skill in the art may realize that the following description is illustrative only and is not in any way limiting. Other embodiments of the present invention may readily suggest themselves to such skilled persons having the benefit of this disclosure. Referring to Figure 1, an ultrasound system 100 in accordance with an illustrative embodiment is shown. As depicted therein, the ultrasound system 100 includes a storage unit 110. The storage unit 110 stores a mapping table including transmit (Tx) frequencies and scan conditions. In one embodiment, the scan conditions include image depths for representing the range of an ultrasound image of the target object to be formed in a depth direction and steering angles for steering a plurality of scan-lines. However, the scan conditions may not be limited thereto.

Figure 2 is a graph showing an example of a grating lobe according to the transmit (Tx) frequencies. Generally, a grating lobe may mean unwanted emission of ultrasound from electronic array transducers at an angle from the main beam. The grating lobe may be stronger as the Tx frequency becomes higher, as shown in Figure 2. Further, the grating lobe may be stronger as the steering angle becomes larger (not shown).

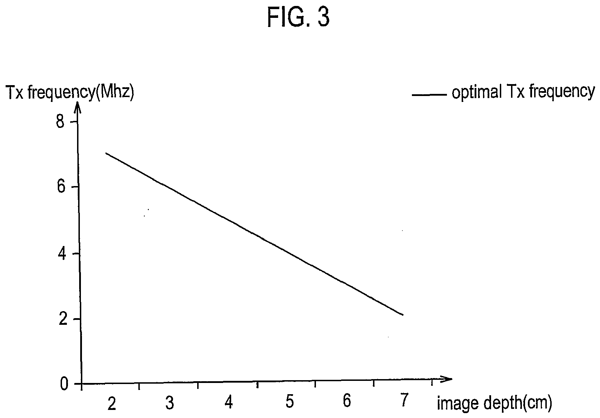

Figure 3 is a graph showing an example of optimal Tx frequencies according to image depths. As shown in Figure 3, a higher Tx frequency is set as the optimal Tx frequency when an image depth is smaller. Also, a lower Tx frequency is set as the optimal frequency when the image depth is larger.

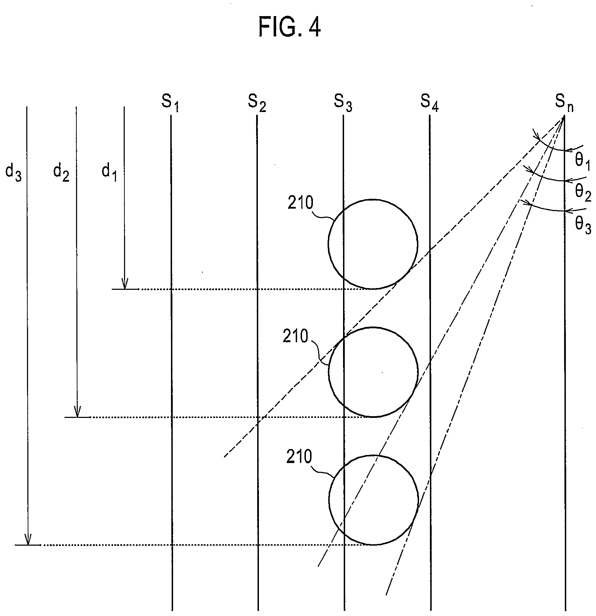

Figure 4 is a schematic diagram showing an example of steering angles set according to the image depths. As shown in Figure 4, a larger steering angle is set when the image depth is smaller. Also, a smaller steering angle is set when the image depth is larger. That is, steering angles θ₁ to θ₃ of scan-lines S₁ to Sₙ is set in consideration of image depths d₁ to d₃ for a target object 210, as shown in Figure 4.

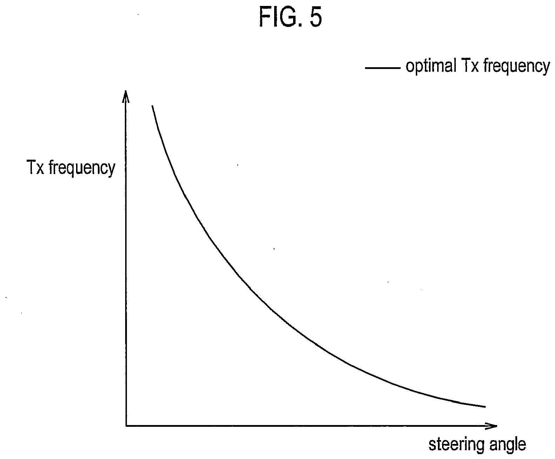

Figure 5 is a graph showing an example of optimal Tx frequencies set according to the steering angles. As shown in Figure 5, a lower Tx frequency is set as the optimal Tx frequency to decrease the grating lobe when a steering angle is larger. Also, a higher Tx frequency is set as the optimal Tx frequency to decrease the grating lobe when the steering angle is smaller, as shown in Figure 5.

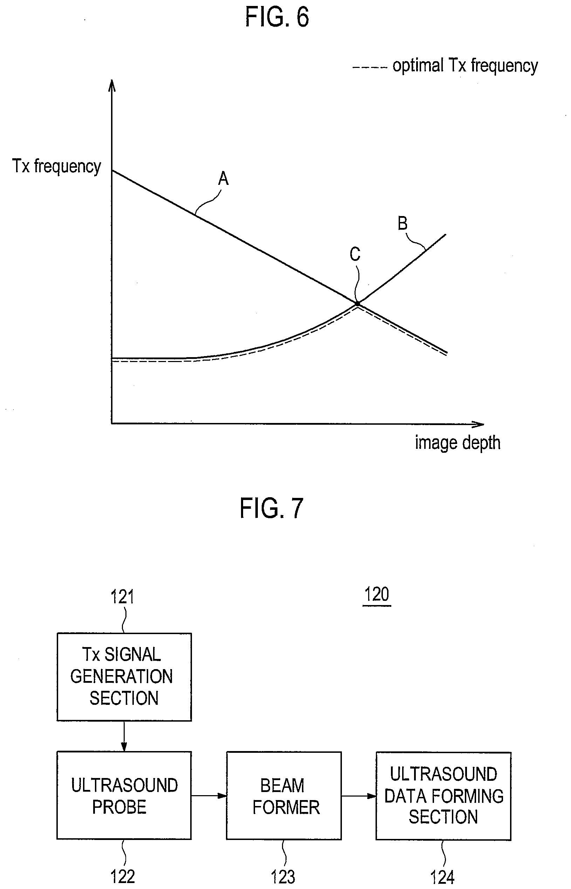

Figure 6 is a schematic diagram showing an example of the optimal Tx frequencies according to scan conditions (i.e., the image depths and steering angles). When only an image depth for a target object is considered to set an optimal Tx frequency as in a conventional method, the resultant values are obtained as indicated in A in Figure 6. Also, when only a steering angle according to the image depth is considered to set an optimal Tx frequency as in the conventional method, the resultant values are obtained as indicated in B in Figure 6. However, in this embodiment, both the image depth and the steering angle are considered to set an optimal Tx frequency.

When an image depth for the target object is less than a predetermined threshold value C (i.e., the target object is located on a near position from a surface of a subject, e.g., a patient), the optimal Tx frequency is set according to the steering angle as shown in Figure 6 to decrease the grating lobe. Also, when the image depth for the target object is more than the predetermined threshold value C (i.e., the target object is located on a far position from the surface of the subject), the optimal Tx frequency is set according to the image depth as shown in Figure 6 to decrease the grating lobe.

In one embodiment, the mapping table is a table for setting the Tx frequency in consideration of the steering angles when the image depth is less than the predetermined threshold value and setting the Tx frequency in consideration of the image depth when the image depth is more than the predetermined threshold value.

Referring back to Figure 1, the ultrasound system further includes an ultrasound data acquisition unit 120. The ultrasound data acquisition unit 120 is configured to transmit and receive ultrasound signals to and from a target object to thereby output ultrasound data.

Figure 7 is a block diagram showing an illustrative embodiment of the ultrasound data acquisition unit 120. Referring to Figure 7, the ultrasound data acquisition unit 120 may include a Tx signal generating section 121, an ultrasound probe 122, a beam former 123 and an ultrasound data forming section 124.

The Tx signal generating section 121 is configured to retrieve a Tx frequency corresponding to a scan condition (i.e., the image depth and steering angle) from the storage unit 110. In one embodiment, the scan condition may be manually set by a user. In another embodiment, the scan condition may be automatically set by the ultrasound system 100. The Tx signal generating section 121 may be further configured to form Tx signals having the retrieved Tx frequency.

The ultrasound probe 122 may include a plurality of elements for reciprocally converting between ultrasound signals and electrical signals. The ultrasound probe 122 is configured to form ultrasound signals having the retrieved Tx frequency in response to the Tx signals to thereby transmit the ultrasound signals into the target object. The ultrasound probe 122 may further receive echo signals reflected from the target object to thereby output received signals. The received signals may be analog signals. The ultrasound probe 122 may include a linear probe, a convex probe, a 3D (three-dimensional) mechanical probe, a 2D (two-dimensional) probe or the like. However, it should be noted herein that the ultrasound probe 122 may not be limited thereto.

The beam former 123 may be configured to convert the received signals into digital signals. The beam former 123 may further perform receiving-focusing upon the digital signals in consideration of distances between the elements and focal points, the image depth and the steering angle to thereby form digital receive-focused signals.

The ultrasound data forming section 124 may be configured to form ultrasound data based on the digital receive-focused signals. The ultrasound data forming section 124 may be further configured to perform various signal processing (e.g., gain adjustment or the like) to the digital receive-focused signals.

Referring back to Figure 1, the ultrasound system may further include a processing unit 130. The processing unit 130 may form an ultrasound image of the target object based on the ultrasound data provided from the ultrasound data acquisition unit 120. The ultrasound image may include a brightness mode image, an elastic image, an ultrasound spatial compound image, a 3D (three-dimensional) ultrasound image or the like. However, the ultrasound image may not be limited thereto.

The ultrasound system may further include a display unit 140. The display unit 140 may display the ultrasound image provided from the processing unit 130.

In another embodiment, the present invention provides a computer readable medium comprising computer executable instructions configured to perform following acts: a) retrieving a Tx frequency corresponding to a scan condition from a mapping table including transmit (Tx) frequencies and scan conditions; and b) forming ultrasound signals having the retrieved Tx frequency. The computer readable medium may comprise a floppy disk, a hard disk, a memory, a compact disk, a digital video disk, etc.

## Claims

1. An ultrasound system (100), comprising:
a storage unit (110) for storing a mapping table including transmit, Tx, frequencies and scan conditions including image depths and steering angles; and
an ultrasound data acquisition unit (120) in communication with the storage unit (110) and being configured to retrieve a Tx frequency corresponding to a scan condition from the storage unit (110) and form ultrasound signals having the retrieved Tx frequency, **characterized in that** the mapping table is a table for setting the Tx frequency in consideration of the steering angles when an image depth of the scan condition is less than a predetermined threshold value and setting the Tx frequency in consideration of the image depths when the image depth of the scan condition is more than the predetermined threshold value, and **in that** a higher Tx frequency is set when the image depth is larger at the image depths less than the predetermined threshold value, and a lower Tx frequency is set when the image depth is larger at the image depths more than the predetermined threshold value.

2. A method of setting a transmit, Tx, frequency, comprising:
a) retrieving a Tx frequency corresponding to a scan condition from a mapping table including Tx frequencies and scan conditions including image depths and steering angles; and
b) forming ultrasound signals having the retrieved Tx frequency, **characterized in that** the mapping table is a table for setting the Tx frequency in consideration of the steering angles when an image depth of the scan condition is less than a predetermined threshold value and setting the Tx frequency in consideration of the image depths when the image depth of the scan condition is more than the predetermined threshold value, and **in that** a higher Tx frequency is set when the image depth is larger at the image depths less than the predetermined threshold value, and a lower Tx frequency is set when the image depth is larger at the image depths more than the predetermined threshold value.

3. A computer readable medium comprising computer executable instructions configured to perform following acts:
a) retrieving a transmit, Tx, frequency corresponding to a scan condition from a mapping table including Tx frequencies and scan conditions including image depths and steering angles; and
b) forming ultrasound signals having the retrieved Tx frequency, **characterized in that** the mapping table is a table for setting the Tx frequency in consideration of the steering angles when an image depth of the scan condition is less than a predetermined threshold value and setting the Tx frequency in consideration of the image depths when the image depth of the scan condition is more than the predetermined threshold value, and **in that** a higher Tx frequency is set when the image depth is larger at the image depths less than the predetermined threshold value, and a lower Tx frequency is set when the image depth is larger at the image depths more than the predetermined threshold value.

## Patentansprüche

1. Ultraschallsystem (100), welches Folgendes aufweist:
eine Speichereinheit (110) zum Speichern einer Mapping-Tabelle, die Übertragungs-Tx-Frequenzen enthält, und Scanzustände bzw. -bedingungen, die Bildtiefen und Steuerwinkel enthalten, und
eine Ultraschalldatenübertragungseinheit (120), die in Verbindung mit der Speichereinheit (110) steht und die dafür vorgesehen ist, eine Tx-Frequenz zu erlangen bzw. abzufragen, die einer Scanbedingung aus der Speichereinheit (110) entspricht, und Ultraschallsignale zu bilden, welche die ermittelten Tx-Frequenzen aufweisen,
**dadurch gekennzeichnet, dass**
die Mapping-Tabelle eine Tabelle zum Festlegen der Tx-Frequenz unter Berücksichtigung der Steuerwinkel, wenn eine Bildtiefe der Scanbedingung weniger als ein vorbestimmter Grenzwert ist, und zum Einstellen der Tx-Frequenz unter Berücksichtigung der Bildtiefen ist, wenn die Bildtiefe der Scanbedingung mehr als der vorbestimmte Grenzwert ist,
und dass eine höhere Tx-Frequenz eingestellt wird, wenn die Bildtiefe an den Bildtiefen um weniger als den vorbestimmten Grenzwert größer ist, und eine niedrigere Tx-Frequenz eingestellt wird, wenn die Bildtiefe an den Bildtiefen um mehr als den vorbestimmten Grenzwert größer ist.

2. Verfahren zum Festlegen einer Übertragungs-Tx-Frequenz, welches Folgendes aufweist:
a) Erlangen bzw. Abfragen einer Tx-Frequenz, die einer Scanbedingung aus einer Mapping-Tabelle, die Tx-Frequenzen enthält, und Scanbedingungen entspricht, die Bildtiefen und Steuerwinkel enthalten; und
b) Bilden von Ultraschallsignalen, welche die erlangten bzw. abgefragten Tx-Frequenzen aufweisen,
**dadurch gekennzeichnet, dass**
die Mapping-Tabelle eine Tabelle zum Festlegen der Tx-Frequenz unter Berücksichtigung der Steuerwinkel, wenn eine Bildtiefe der Scanbedingung weniger als ein vorbestimmter Grenzwert ist, und zum Einstellen der Tx-Frequenz unter Berücksichtigung der Bildtiefen ist, wenn die Bildtiefe der Scanbedingung mehr als der vorbestimmte Grenzwert ist,
und dass eine höhere Tx-Frequenz eingestellt wird, wenn die Bildtiefe an den Bildtiefen um weniger als den vorbestimmten Grenzwert größer ist, und eine niedrigere Tx-Frequenz eingestellt wird, wenn die Bildtiefe an den Bildtiefen um mehr als den vorbestimmten Grenzwert größer ist.

3. Computerlesbares Medium, welches von einem Computer ausführbare Befehle aufweist, das dafür vorgesehen ist, die folgende Vorgänge durchzuführen:
a) Erlangen bzw. Abfragen einer Übertragungs-Tx-Frequenz, die einer Scanbedingung aus einer Mapping-Tabelle, die Tx-Frequenzen enthält, und Scanbedingungen entspricht, die Bildtiefen und Steuerwinkel enthalten; und
b) Bilden von Ultraschallsignalen, welche die erlangten bzw. abgefragten Tx-Frequenzen aufweisen,
**dadurch gekennzeichnet, dass**
die Mapping-Tabelle eine Tabelle zum Festlegen der Tx-Frequenz unter Berücksichtigung der Steuerwinkel, wenn eine Bildtiefe der Scanbedingung weniger als ein vorbestimmter Grenzwert ist, und zum Einstellen der Tx-Frequenz unter Berücksichtigung der Bildtiefen ist, wenn die Bildtiefe der Scanbedingung mehr als der vorbestimmte Grenzwert ist,
und dass eine höhere Tx-Frequenz eingestellt wird, wenn die Bildtiefe an den Bildtiefen um weniger als den vorbestimmten Grenzwert größer ist, und eine niedrigere Tx-Frequenz eingestellt wird, wenn die Bildtiefe an den Bildtiefen um mehr als den vorbestimmten Grenzwert größer ist.

## Revendications

1. Système ultrasonique (100) comportant :
une unité de mémorisation (110) pour stocker une table de modélisation comportant, la transmission, les fréquences Tx et des conditions de balayage comportant des profondeurs d'image et des angles de directions ; et
une unité d'acquisitions de données ultrasoniques (120) en communication avec l'unité de mémorisation (110) et étant configurée pour récupérer une fréquence Tx correspondant à une condition de balayage de l'unité de mémorisation (110) et formant des signaux ultrasoniques ayant la fréquence Tx récupérée ;
**caractérisé en ce que**
la table de modélisation est une table pour placer la fréquence Tx en considération avec les angles de directions, lorsque la profondeur d'image de la condition de balayage est inférieure à une valeur prédéterminée d'un seuil et fixer la fréquence Tx en considération des profondeurs d'image lorsque la profondeur d'image de la condition de balayage est supérieure à une valeur prédéterminée de seuil, et **en ce que**
une fréquence Tx plus élevée est définie lorsque la profondeur d'image est plus grande que les profondeurs d'images inférieures à la valeur du seuil prédéterminé, et une fréquence Tx plus basse est fixée lorsque la profondeur d'image est plus importante que les profondeurs d'image supérieures à la valeur prédéterminée de seuil.

2. Procédé pour effectuer un transfert, Tx, fréquence comportant :
a) la récupération d'une fréquence Tx correspondant à une condition de balayage d'une table de modélisation de fréquences Tx et les conditions de balayage comportant les profondeurs d'image et les angles de direction ; et
b) la formation de signaux ultrasoniques ayant la fréquence Tx récupérée,
**caractérisé en ce que**
la table de modélisation est une table pour placer les fréquences Tx en considération des angles de direction, lorsque une profondeur d'image des conditions de balayage est inférieure à une valeur prédéterminée d'un seuil, et la fixation de la fréquence Tx en considération des profondeurs d'image, lorsque la profondeur d'image de la condition de balayage est supérieure à une valeur prédéterminée de seuil, et **en ce**
**qu'**une fréquence Tx supérieure est fixée lorsque la profondeur d'image est supérieure aux profondeurs d'image inférieures à la valeur prédéterminée de seuil, et une fréquence Tx plus basse est fixée lorsque la profondeur d'image est plus grande que les profondeurs d'images supérieures à la valeur prédéterminée de seuil.

3. Support lisible par un ordinateur, comportant des instructions pouvant être exécutées par l'ordinateur pour effectuer les actions suivantes :
a) la récupération d'une fréquence Tx correspondant à une condition de balayage d'une table de modélisation de fréquences Tx et les conditions de balayage comportant les profondeurs d'image et les angles de direction ; et
b) la formation de signaux ultrasoniques ayant la fréquence Tx récupérée,
**caractérisé en ce que**
la table de modélisation est une table pour placer les fréquences Tx en considération des angles de direction, lorsque une profondeur d'image des conditions de balayage est inférieure à une valeur prédéterminée d'un seuil, et la fixation de la fréquence Tx en considération des profondeurs d'image, lorsque la profondeur d'image de la condition de balayage est supérieure à une valeur prédéterminée de seuil, et **en ce**
**qu'**une fréquence Tx supérieure est fixée lorsque la profondeur d'image est supérieure aux profondeurs d'image inférieures à la valeur prédéterminée de seuil, et une fréquence Tx plus basse est fixée lorsque la profondeur d'image est plus grande que les profondeurs d'images supérieures à la valeur prédéterminée de seuil.
